# FASCICULE DE BREVET EUROPEEN

(11) **EP 4 188 253 B1**
(45) Date de publication et mention de la délivrance du brevet: **04.09.2024**
(21) Numéro de dépôt: 21746066.6
(22) Date de dépôt: 26.07.2021
(51) Int. Cl.: A61B 17/80, A61B 17/72, A61B 17/86, A61B 17/84

(54) **DISPOSITIF D'OSTÉOSYNTHÈSE COMPRENANT AU MOINS UNE BROCHE DE FIXATION**
OSTEOSYNTHESEVORRICHTUNG MIT MINDESTENS EINEM FIXATIONSSTIFT
OSTEOSYNTHESIS DEVICE COMPRISING AT LEAST ONE FIXATION PIN

(30) Priorité: 27.07.2020 FR 2007917
(43) Date de publication de la demande: 07.06.2023
(73) Titulaire: Newclip International, 2163 Luxembourg (LU)
(72) Inventeur: BALLERINI, Julien, 44100 NANTES (FR); PODGORSKI, Jean-Pierre, 49230 SAINT CRESPIN SUR MOINE (FR); LARCHE, Grégoire, 49300 CHOLET (FR); ROSS, Mark, BRISBANE, Queensland 4000 (AU)
(74) Mandataire: Jacobacci Coralis Harle
(86) Numéro de dépôt international: PCT/EP2021/070833
(87) Numéro de publication internationale: WO 2022/023252

(56) Documents cités:
- WO-A1-2018/187770
- FR-A1- 2 905 589
- US-A1- 2005 154 392
- US-A1- 2007 010 817
- US-A1- 2011 264 149
- US-A1- 2016 157 903

## Description

### Domaine technique de l'invention

La présente invention concerne le domaine général du matériel chirurgical destiné aux techniques d'ostéosynthèse.

Elle concerne plus particulièrement les dispositifs d'ostéosynthèse comprenant une ou plusieurs broches de fixation adaptées pour la réduction d'une fracture osseuse.

### Etat de la technique

La réduction et la stabilisation des fractures osseuses sont généralement réalisées au moyen de vis de fixation et/ou de broches de fixation implantées dans le matériau osseux, éventuellement en association avec une plaque d'ostéosynthèse.

Les broches de fixation utilisées se présentent sous la forme de tiges métalliques lisses ou filetées, dont l'une au moins des extrémités est pointue ou effilée pour permettre son enfoncement dans le matériau osseux, généralement au moyen d'un moteur chirurgical. Elles sont généralement utilisées pour stabiliser les fragments osseux de petites dimensions.

Cependant, après enfoncement, ces broches peuvent migrer au sein du matériau osseux de réception, dans un sens ou dans l'autre, en fonction des sollicitations.

Cette possibilité de mouvement est susceptible de poser des problèmes de stabilisation de la réduction et peut causer des dommages aux tissus environnants.

Pour chercher à limiter cette possibilité de déplacement, après positionnement de la broche, le chirurgien peut replier son extrémité saillante contre le matériau osseux ou contre la plaque d'ostéosynthèse associée.

Cependant une telle opération de repliement ou de cintrage n'est pas facile à réaliser ; de plus elle ne peut empêcher la migration de la broche que dans un seul sens.

### Présentation de l'invention

Afin de remédier à cet inconvénient de l'état de la technique, la présente invention propose un dispositif d'ostéosynthèse comprenant au moins une broche de fixation adaptée pour être implantée dans un matériau osseux de sorte à assurer une réduction au moins partielle d'une fracture osseuse, ce dispositif d'ostéosynthèse comprenant des moyens de verrouillage agencés pour verrouiller en position ladite au moins une broche de fixation implantée dans ledit matériau osseux.

La présente invention est définie dans la revendication 1, tandis que les modes de réalisation préférés sont exposés dans les revendications dépendantes.

Plus particulièrement, les moyens de verrouillage du dispositif d'ostéosynthèse selon l'invention peuvent comprendre :
(a) une structure support munie de moyens qui permettent sa fixation sur le matériau osseux, laquelle structure support comprend un orifice de verrouillage qui traverse son épaisseur, lequel orifice de verrouillage est centré sur un axe d'orifice et est délimité par un contour d'orifice, lequel orifice de verrouillage est adapté pour être traversé par ladite au moins une broche de fixation associée, et
(b) un moyen de serrage agencé pour être rapporté dans ledit orifice de verrouillage et pour venir prendre appui contre une partie de ladite au moins une broche de fixation afin d'assurer son verrouillage en position.

Selon une forme de réalisation préférée, une partie au moins dudit contour d'orifice de l'orifice de verrouillage est munie d'un taraudage d'orifice,
et ledit moyen de serrage consiste en un écrou de verrouillage centré sur un axe d'écrou, lequel écrou de verrouillage est délimité par - une partie supérieure d'écrou munie d'une empreinte adaptée pour sa manoeuvre en rotation au moyen d'un outil approprié, - une partie inférieure d'écrou située à l'opposé de ladite partie supérieure d'écrou, - une enveloppe périphérique externe comprenant un filetage d'écrou adapté à coopérer avec ledit taraudage d'orifice dudit orifice de verrouillage de la structure support, et - un contour intérieur délimitant une lumière axiale adaptée au passage de ladite au moins une broche de fixation,
lequel écrou de verrouillage comprend encore une structure de serrage déformable adaptée pour se déformer lorsque ledit écrou de verrouillage est vissé dans ledit taraudage d'orifice et pour venir en appui contre une partie de ladite au moins une broche de fixation logée au sein de ladite lumière axiale.

D'autres caractéristiques non limitatives et avantageuses du dispositif d'ostéosynthèse conforme à l'invention, prises individuellement ou selon toutes les combinaisons techniquement possibles, sont les suivantes :
- la structure de serrage comprend au moins une patte de serrage ménagée au niveau de la partie inférieure d'écrou ;
- ladite au moins une patte de serrage est déformable par pliage autour d'un axe de pliage perpendiculaire audit axe d'écrou ;
- la structure de serrage comprend une pluralité de pattes de serrage régulièrement réparties sur le pourtour de la partie inférieure d'écrou, chacune desdites patte de serrage étant déformable par pliage autour d'un axe de pliage perpendiculaire audit axe d'écrou, les différents axes de pliages desdites pattes de serrage étant situés dans un même plan perpendiculaire audit axe d'écrou ;
- la structure de serrage comprend 2 à 4 pattes de serrage, de préférence 3 ;
- la structure de serrage comprend une pluralité de pattes de serrage séparées les unes des autres par des fentes s'étendant dans des plans radiaux par rapport audit axe d'écrou ;
- le contour d'orifice de l'orifice de verrouillage de la structure support est de forme générale tronconique, l'angle dudit tronc de cône par rapport à l'axe d'orifice de l'orifice de verrouillage étant compris entre 9° et 11° ;
- l'enveloppe périphérique externe de l'écrou de verrouillage constituant le moyen de serrage est de forme générale tronconique, l'angle dudit tronc de cône par rapport à l'axe d'écrou de l'écrou de verrouillage étant compris entre 7° et 9° ;
- les moyens qui permettent la fixation de la structure support sur le matériau osseux consistent en au moins un orifice de fixation ménagé sur ladite structure support, coopérant avec une vis de fixation ;
- la structure support se présente sous la forme d'une plaque d'ostéosynthèse comprenant (a) une pluralité d'orifices de fixation, et (b) une pluralité d'orifices de verrouillage adaptés au moins pour certains à l'accueil d'une broche de fixation.

De préférence, les orifices de fixation et les orifices de verrouillage de la plaque d'ostéosynthèse sont identiques pour pouvoir accueillir chacun, au choix, une vis de fixation ou une broche de fixation.

Bien entendu, les différentes caractéristiques, variantes et formes de réalisation de l'invention peuvent être associées les unes avec les autres selon diverses combinaisons dans la mesure où elles ne sont pas incompatibles ou exclusives les unes des autres.

### Description détaillée de l'invention

De plus, diverses autres caractéristiques de l'invention ressortent de la description annexée effectuée en référence aux dessins qui illustrent une forme, non limitative, de réalisation de l'invention et où :
[Fig. 1] est une vue schématique, en coupe, montrant un dispositif d'ostéosynthèse conforme à l'invention, avant le vissage de l'écrou de verrouillage dans son orifice de réception de la structure support pour verrouiller en position la broche de fixation ;
[Fig. 2] est une vue schématique en coupe, similaire à la figure 1, illustrant le dispositif d'ostéosynthèse selon l'invention en position activée, après le vissage et le serrage de l'écrou de verrouillage dans son orifice de réception de la structure support pour verrouiller en position la broche de fixation ;
[Fig. 3] est une vue en perspective de l'écrou de verrouillage du dispositif d'ostéosynthèse selon l'invention, isolé, vu du côté de sa partie supérieure d'écrou ;
[Fig. 4] est une vue en perspective de l'écrou de verrouillage de la figure 3, vu du côté de sa partie inférieure d'écrou ;
[Fig. 5] est une première vue de côté de l'écrou de verrouillage illustré sur les figures 3 et 4 ;
[Fig. 6] est une deuxième vue de côté de l'écrou de verrouillage illustré sur les figures 3 et 4 ;
[Fig. 7] est une vue de dessus de l'écrou de verrouillage illustré sur les figures 3 à 6 ;
[Fig. 8] montre l'écrou de verrouillage du dispositif d'ostéosynthèse selon l'invention, au travers de la lumière centrale duquel s'étend une broche de fixation, vus en perspective du côté de la partie inférieure d'écrou ;
[Fig. 9] est une vue similaire à la figure 8 illustrant l'écrou de verrouillage et la broche de fixation associée selon une perspective du côté de la partie supérieure d'écrou ;
[Fig. 10] est une vue en perspective qui illustre un dispositif d'ostéosynthèse selon l'invention dont la structure support est en forme de plaque d'ostéosynthèse, en place sur un matériau osseux de réception ;
[Fig. 11] est une vue agrandie d'une partie de la figure 10.

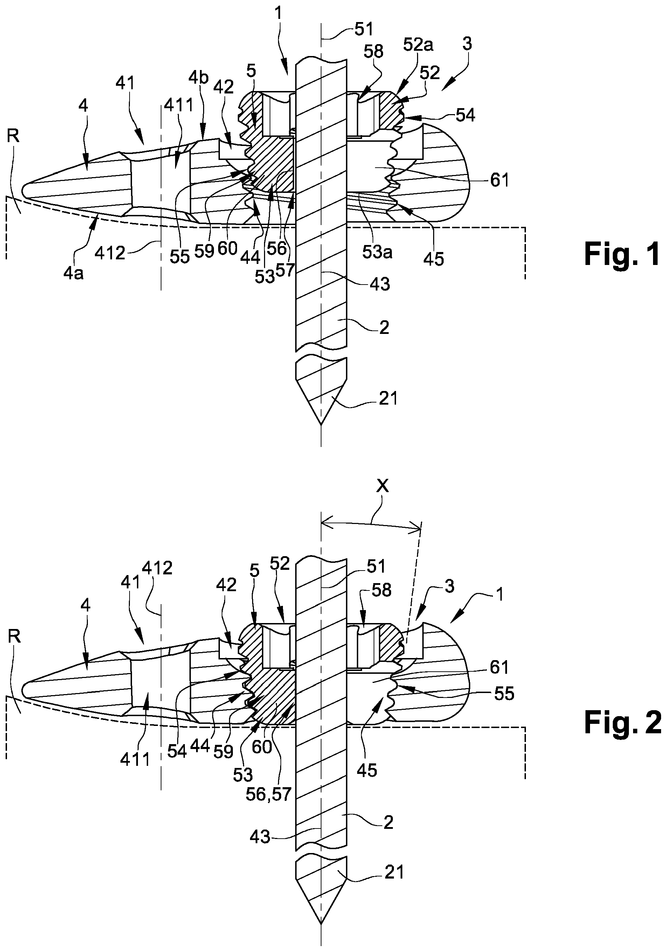

Le dispositif d'ostéosynthèse 1 illustré sur les figures 1 et 2 comprend une broche de fixation 2 adaptée pour être implantée dans un matériau osseux R de sorte à assurer une réduction au moins partielle d'une fracture osseuse, et il comprend également des moyens de verrouillage 3 agencés pour verrouiller en position cette broche de fixation 2.

La broche de fixation 2 est réalisée en métal, par exemple en inox, en titane ou en chrome cobalt. Elle se présente ici sous la forme d'une tige lisse dont au moins une des extrémités 21 est en forme de pointe ; sa longueur peut être comprise entre 20 et 200mm et son diamètre, constant, peut être compris entre 0,8 et 4mm.

Les moyens de verrouillage 3 comprennent :
(a) une structure support 4 munie de moyens 41 qui permettent sa fixation sur le matériau osseux R, et comprenant un orifice de verrouillage 42 qui traverse son épaisseur, lequel orifice de verrouillage 42 est centré sur un axe d'orifice 43 et est délimité par un contour d'orifice 44, lequel orifice de verrouillage 42 est adapté pour être traversé par la broche de fixation 2, et
(b) un moyen de serrage 5 agencé pour être rapporté dans ledit orifice de verrouillage 42 et pour venir prendre appui contre une partie de ladite broche de fixation 2 afin d'assurer son verrouillage en position.

Le contour d'orifice 44 comprend un taraudage d'orifice 45, et le moyen de serrage 5 consiste en un écrou de verrouillage 5 centré sur un axe d'écrou 51.

La structure support 4 se présente ici sous la forme d'une plaque support d'ostéosynthèse dont l'épaisseur est de quelques millimètres et qui est délimitée par une face inférieure 4a destinée à venir en contact avec le matériau osseux R de réception et par une face supérieure 4b opposée.

Outre l'orifice de verrouillage 42 précité des moyens de verrouillage 3, adapté à recevoir la broche de fixation 2 et le moyen de serrage 5, la structure support 4 est munie d'au moins un orifice 411 qui traverse son épaisseur, entre sa face inférieure 4a et sa face supérieure 4b ; cet orifice 411 est destiné au passage d'un organe de fixation 412 (en particulier sous la forme d'une vis de fixation représentée de manière schématique sur les figures 1 et 2) pour sa fixation sur le matériau osseux R de réception.

L'orifice 411, et l'organe de fixation 412 associé, forment les moyens 41 permettant la fixation de la plaque d'ostéosynthèse 4 sur le matériau osseux R.

Le contour d'orifice 44 de l'orifice de verrouillage 42 de la structure support 4 est de forme générale tronconique dont le sommet est orienté du côté de la face inférieure 4a de la structure support 4. L'angle X de ce tronc de cône par rapport à l'axe d'orifice 43 est compris entre 5° et 15° (de préférence cet angle X est compris entre 8° et 12°, et encore de préférence entre 9° et 11°).

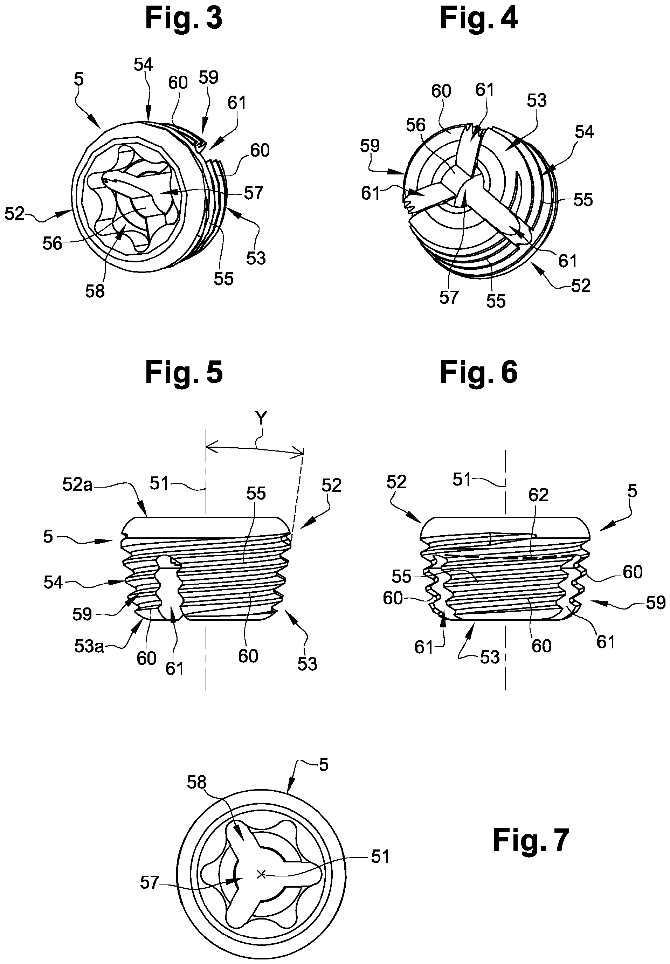

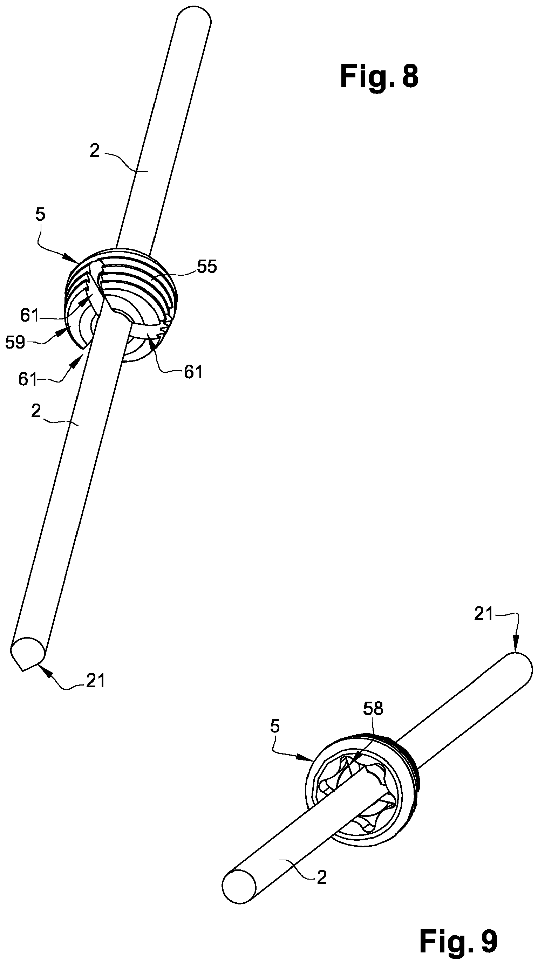

L'écrou de verrouillage 5 est illustré isolément sur les figures 3 à 7, et en association avec une broche de fixation 2 sur les figures 8 et 9.

Cet écrou de verrouillage 5 est délimité par - une partie supérieure d'écrou 52, comprenant une extrémité supérieure d'écrou 52a, - une partie inférieure d'écrou 53, comprenant une extrémité inférieure d'écrou 53a, située à l'opposé de ladite partie supérieure d'écrou 52, - une enveloppe périphérique externe 54 comprenant un filetage d'écrou 55 adapté à coopérer avec le taraudage d'orifice 45 de l'orifice de verrouillage 42, et - un contour intérieur 56 délimitant une lumière axiale 57 adaptée au passage de la broche de fixation 2.

La partie supérieure d'écrou 52 est munie d'une empreinte 58 adaptée pour sa manoeuvre en rotation au moyen d'un outil approprié, en vue du vissage ou du dévissage de l'écrou de verrouillage 5.

L'enveloppe périphérique externe 54 de l'écrou de verrouillage 5 est de forme générale tronconique dont le sommet est orienté du côté de la partie inférieure d'écrou 53. L'angle Y de cette enveloppe périphérique externe 54 en forme de tronc de cône est compris entre 4° et 12° (de préférence cet angle Y est compris entre 6° et 10°, et encore de préférence entre 7° et 9°).

L'écrou de verrouillage 5 comprend encore une structure de serrage 59 déformable, adaptée pour se déformer lorsque ledit écrou de verrouillage 5 est vissé dans le taraudage d'orifice 45, et pour venir en appui contre une partie de la broche de fixation 2 logée au sein de la lumière axiale 57.

Cette structure de serrage 59 comprend au moins une patte de serrage 60, et de préférence plusieurs pattes de serrage 60, ménagée(s) au niveau de la partie inférieure d'écrou 53.

En l'occurrence la structure de serrage 59 comprend une pluralité de pattes de serrage 60 régulièrement réparties sur le pourtour de la partie inférieure d'écrou 53. Ces pattes de serrage 60 sont séparées les unes des autres par des fentes 61 qui s'étendent dans des plans radiaux par rapport à l'axe d'écrou 51 ; et chacune de ces pattes de serrage 60 est déformable par pliage autour d'un axe de pliage perpendiculaire audit axe d'écrou 51.

Les fentes 61 s'étendent depuis l'extrémité inférieure d'écrou 53a, sur une partie de la hauteur de l'écrou de verrouillage 5, en l'occurrence, ici, sur un peu plus de la moitié de la hauteur de l'écrou de verrouillage 5.

L'axe de pliage correspondant est illustré sur la figure 6 par le repère 62.

Les différents axes de pliages 62 des pattes de serrage 60 sont situés dans un même plan perpendiculaire audit axe d'écrou 51.

De préférence les pattes de serrage 60 sont au nombre de 2, 3 ou 4. Dans le mode de réalisation illustré, la structure de serrage 59 comprend 3 pattes de serrage 60.

Les fentes 61 radiales réalisées dans la partie inférieure d'écrou 53 définissent les pattes de serrage 60, déformables chacune autour d'un axe de pliage 62 perpendiculaire à l'axe d'écrou 51. Pour chaque patte de serrage 60, l'axe de pliage 62 correspond à la ligne qui raccorde le fond des deux fentes 61 radiales disposées de part et d'autre.

Le filetage d'écrou 55 s'étend sur toute ou pratiquement toute la hauteur de l'enveloppe périphérique externe 54, entre la partie supérieure d'écrou 52 et la partie inférieure d'écrou 53.

Les pattes de serrage 60 font partie intégrante de l'écrou de verrouillage 5. Leur contour externe s'étend sur l'enveloppe périphérique externe 54 de forme tronconique, et ce contour externe des pattes de serrage 60 comprend une partie du filetage d'écrou 55.

La forme de l'orifice de verrouillage 42 est adaptée pour appliquer une force d'appui radiale sur le contour de la partie inférieure 53 de l'écrou de verrouillage 5 au fur et à mesure du vissage de ce dernier sur le taraudage d'orifice 45, de sorte à obtenir la fonction de serrage recherchée.

Pour cela, avant vissage, le diamètre de la partie inférieure de l'orifice de verrouillage 42 est inférieur au diamètre de l'extrémité inférieure 53a de l'écrou de verrouillage 5.

Avant activation de la structure de serrage 59, la lumière axiale 57 de l'écrou de verrouillage 5 permet le passage en son travers d'une broche de fixation 2.

La lumière axiale 57 peut alors se présenter sous la forme d'un canal ou d'un orifice cylindrique dont le diamètre correspond, au jeu près, au diamètre de la broche de fixation 2.

Cette possibilité de passage ou de coulissement est illustrée sur la figure 1, qui montre l'écrou de verrouillage 5 avant son serrage dans l'orifice de verrouillage 42, par le petit espace présent entre le contour intérieur 56 de l'écrou de verrouillage 5 (qui délimite la lumière axiale 57) et la paroi cylindrique en regard de la broche de fixation 2.

Sur les figures 8 et 9, l'écrou de verrouillage 5 n'est pas introduit dans un orifice de verrouillage 42 d'une structure support 4, et la broche de fixation 2 peut également coulisser librement dans la lumière axiale 57 dudit écrou de verrouillage 5.

Une fois la structure support 4 fixée sur un matériau osseux de réception R, avec l'écrou de verrouillage 5 non vissé ou non complètement vissé dans l'orifice de verrouillage 42 (tel qu'illustré sur la figure 1 par exemple), la broche de fixation 2 peut être mise en place par le chirurgien, en passant au travers de la lumière axiale 57 de l'écrou de verrouillage 5, de manière à réaliser la réduction recherchée de la fracture osseuse.

Lorsque la broche de fixation 2 est correctement positionnée au sein du matériau osseux R, son positionnement peut être verrouillé par vissage de l'écrou de verrouillage 5 sur le taraudage de l'orifice de verrouillage 42 de la structure support 4.

Lors de ce vissage, la forme adaptée de l'orifice de verrouillage 42 force les pattes de serrage 60 de l'écrou de verrouillage 5 à se déformer radialement en direction de l'axe d'écrou 51, et donc en direction de la broche de fixation 2.

Cette déformation s'effectue par pliage des pattes de serrage 60 autour de leur axe de pliage 62 respectif, jusqu'à ce que ces pattes de serrage 60 viennent en butée contre la broche de fixation 2.

On comprend alors qu'un vissage à force de l'écrou de verrouillage 5 permet aux pattes de serrage 60 d'appliquer une force d'appui importante contre la broche de fixation 2, assurant le verrouillage en position de cette broche de fixation 2 qui ne peut plus coulisser, ou migrer, ni dans le sens dans l'autre, tel qu'illustré sur la figure 2.

La pluralité de pattes de serrage 60 régulièrement réparties sur la périphérie de la partie inférieure d'écrou 53 permet d'obtenir un serrage homogène de la broche de fixation 2.

L'écrou de verrouillage 5 fait en quelque sorte office de coin de verrouillage entre le contour d'orifice 44 et la broche de fixation 2.

La forme du contour d'orifice 44 de l'orifice de verrouillage 42 (angle X) et la forme de l'enveloppe périphérique externe 54 de l'écrou de verrouillage 5 (angle Y), ainsi que leurs dimensions, sont adaptées pour obtenir la déformation des pattes de serrage 60 et le verrouillage recherché, lorsque l'écrou de verrouillage 5 est vissé dans l'orifice de verrouillage 42

Dans le mode de réalisation illustré le contour d'orifice 44 de l'orifice de verrouillage 42 et l'enveloppe périphérique externe 54 de l'écrou de verrouillage 5 ont tous deux une forme générale tronconique, mais dont l'angle au sommet est différent ; dans une variante de réalisation l'enveloppe périphérique externe de l'écrou de verrouillage peut être de forme générale cylindrique, le contour d'orifice de l'orifice de verrouillage de la structure support ayant de son côté de forme générale tronconique.

Encore à titre de variante, d'autres moyens qu'un écrou de verrouillage peuvent être utilisés pour bloquer la broche de fixation par rapport à la structure support 4. Par exemple on peut prévoir une couronne conique, comprenant au moins une partie souple, adaptée pour pouvoir être impactée entre le contour d'orifice 44 et la broche de fixation 2, formant ainsi une sorte de coin de verrouillage.

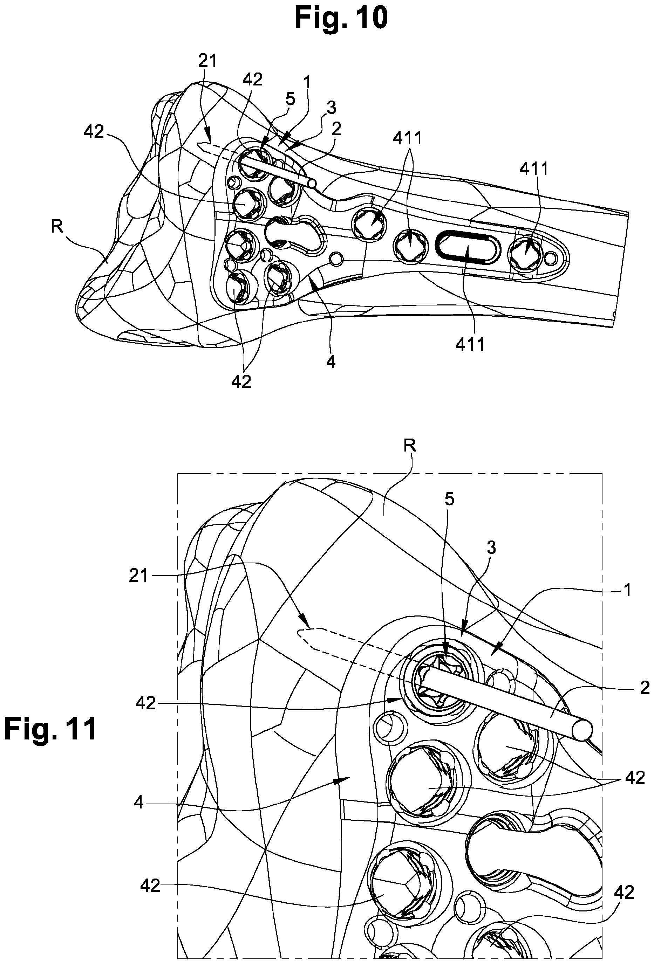

Comme illustré sur les figures 10 et 11, la structure support 4 peut se présenter sous la forme d'une plaque d'ostéosynthèse comprenant une pluralité d'orifices de fixation 411, pour sa fixation sur le matériau osseux R, et une pluralité d'orifices de verrouillage 42 adapté chacun à l'accueil d'une broche de fixation 2.

Dans une forme de réalisation particulière, les orifices de fixation pour la fixation sur le matériau osseux R, et les orifices de verrouillage pour l'accueil des broches de fixation 2, de la plaque d'ostéosynthèse 4, sont prévus identiques afin de pouvoir accueillir chacun, au choix, une vis de fixation 412 ou une broche de fixation 2.

On comprend que le chirurgien peut réduire une fracture osseuse au moyen d'une ou de plusieurs broches de fixation 2, de manière classique, et verrouiller en position chacune de ces broches de fixation lorsqu'il estime que leur position est correcte.

Les broches de fixation 2 peuvent être fixées temporairement ou à demeure dans le matériau osseux de réception.

Après une fixation temporaire, le chirurgien dévisse l'écrou de verrouillage 5 ; et la broche de fixation 2 retrouve alors sa possibilité de coulissement dans la lumière axiale 57 de l'écrou de verrouillage 5, en vue de son enlèvement.

Lors d'une fixation durable, le chirurgien coupe la broche de fixation 2 au ras de la plaque support pour éviter la présence d'une partie saillante.

## Revendications

1. Dispositif d'ostéosynthèse comprenant au moins une broche de fixation (2) adaptée pour être implantée dans un matériau osseux (R) de sorte à assurer une réduction au moins partielle d'une fracture osseuse,
lequel dispositif d'ostéosynthèse comprend des moyens de verrouillage (3) agencés pour verrouiller en position ladite au moins une broche de fixation (2) implantée dans ledit matériau osseux (R),
lesquels moyens de verrouillage (3) comprennent :
(a) une structure support (4) munie de moyens (41) qui permettent sa fixation sur le matériau osseux (R), laquelle structure support (4) comprend un orifice de verrouillage (42) qui traverse son épaisseur, lequel orifice de verrouillage (42) est centré sur un axe d'orifice (43) et est délimité par un contour d'orifice (44), dont une partie au moins est munie d'un taraudage d'orifice (45), lequel orifice de verrouillage (42) est adapté pour être traversé par ladite au moins une broche de fixation (2), et
(b) un moyen de serrage (5) agencé pour être rapporté dans ledit orifice de verrouillage (42) et pour venir prendre appui contre une partie de ladite au moins une broche de fixation (2) afin d'assurer son verrouillage en position,
lequel moyen de serrage (5) consiste en un écrou de verrouillage (5) centré sur un axe d'écrou (51), lequel écrou de verrouillage (5) est délimité par - une partie supérieure d'écrou (52) munie d'une empreinte (58) adaptée pour sa manoeuvre en rotation au moyen d'un outil approprié, - une partie inférieure d'écrou (53) située à l'opposé de ladite partie supérieure d'écrou (52), - une enveloppe périphérique externe (54) comprenant un filetage d'écrou (55) adapté à coopérer avec ledit taraudage d'orifice (45) dudit orifice de verrouillage (42), et - un contour intérieur (56) délimitant une lumière axiale (57) adaptée au passage de ladite au moins une broche de fixation (2),
**caractérisé en ce que** ledit écrou de verrouillage (5) comprend encore une structure de serrage (59) déformable conçue pour se déformer lorsque ledit écrou de verrouillage (5) est vissé dans ledit taraudage d'orifice (45) et pour venir en appui contre une partie de ladite au moins une broche de fixation (2) logée au sein de ladite lumière axiale (57).

2. Dispositif d'ostéosynthèse selon la revendication 1, **caractérisé en ce que** ladite structure de serrage (59) comprend au moins une patte de serrage (60) ménagée au niveau de ladite partie inférieure d'écrou (53).

3. Dispositif d'ostéosynthèse selon la revendication 2, **caractérisé en ce que** ladite au moins une patte de serrage (60) est déformable par pliage autour d'un axe de pliage (62) perpendiculaire audit axe d'écrou (51).

4. Dispositif d'ostéosynthèse selon l'une quelconque des revendications 2 ou 3, **caractérisé en ce que** ladite structure de serrage (59) comprend une pluralité de pattes de serrage (60) régulièrement réparties sur le pourtour de ladite partie inférieure d'écrou (53), chacune desdites patte de serrage (60) étant déformable par pliage autour d'un axe de pliage (62) perpendiculaire audit axe d'écrou (51), les différents axes de pliages (62) desdites pattes de serrage (60) étant situés dans un même plan perpendiculaire audit axe d'écrou (51).

5. Dispositif d'ostéosynthèse selon l'une quelconque des revendications 2 à 4, **caractérisé en ce que** ladite structure de serrage (59) comprend 2 à 4 pattes de serrage (60), de préférence 3.

6. Dispositif d'ostéosynthèse selon l'une quelconque des revendications 2 à 5, **caractérisé en ce que** ladite structure de serrage (59) comprend une pluralité de pattes de serrage (60) séparées les unes des autres par des fentes (61) s'étendant dans des plans radiaux par rapport audit axe d'écrou (51).

7. Dispositif d'ostéosynthèse selon l'une quelconque des revendications 1 à 6, **caractérisé en ce que** le contour d'orifice (44) de l'orifice de verrouillage (42) de la structure support (4) est de forme générale tronconique, l'angle (X) dudit tronc de cône par rapport à l'axe d'orifice (43) dudit orifice de verrouillage (42) étant compris entre 5° et 15°.

8. Dispositif d'ostéosynthèse selon la revendication 7, **caractérisé en ce que** l'angle (X) dudit tronc de cône par rapport à l'axe d'orifice (43) dudit orifice de verrouillage (42) étant compris entre 9° et 11°.

9. Dispositif d'ostéosynthèse selon l'une quelconque des revendications 1 à 8, **caractérisé en ce que** l'enveloppe périphérique externe (54) de l'écrou de verrouillage (5) constituant ledit moyen de serrage (5) est de forme générale tronconique, l'angle (Y) dudit tronc de cône par rapport à l'axe d'écrou (51) dudit écrou de verrouillage (5) étant compris entre 4° et 12°.

10. Dispositif d'ostéosynthèse selon la revendication 9, **caractérisé en ce que** l'angle (Y) dudit tronc de cône par rapport à l'axe d'écrou (51) dudit écrou de verrouillage (5) étant compris entre 7° et 9°.

11. Dispositif d'ostéosynthèse selon l'une quelconque des revendications 1 à 10, **caractérisé en ce que** lesdits moyens (41) qui permettent la fixation de la structure support (4) sur le matériau osseux (R) consistent en au moins un orifice de fixation (411) ménagé sur ladite structure support (4), coopérant avec une vis de fixation (412).

12. Dispositif d'ostéosynthèse selon la revendication 11, **caractérisé en ce que** ladite structure support (4) se présente sous la forme d'une plaque d'ostéosynthèse (4) comprenant une pluralité d'orifices de fixation (411) et une pluralité d'orifices de verrouillage (42) adapté au moins pour certains à l'accueil d'une broche de fixation (2).

13. Dispositif d'ostéosynthèse selon la revendication 12, **caractérisé en ce que** lesdits orifices de fixation (411) et lesdits orifices de verrouillage (42) de la plaque d'ostéosynthèse (4) sont identiques pour pouvoir accueillir chacun, au choix, une vis de fixation (412) ou une broche de fixation (2).

## Patentansprüche

1. Osteosynthesevorrichtung mit mindestens einem Befestigungsstift (2), der dazu ausgelegt ist, in knochenartiges Material (R) eingesetzt zu werden, um eine mindestens teilweise Reduzierung eines Knochenbruchs sicherzustellen,
wobei die Osteosynthesevorrichtung Verriegelungsmittel (3) aufweist, die dazu ausgelegt sind, den in das knochenartige Material (R) eingesetzten Befestigungsstift (2) in der Position zu verriegeln,
wobei die Verriegelungsmittel (3)
(a) eine Trägerstruktur (4) aufweisen, die mit Mitteln (41) versehen ist, die deren Befestigung auf dem knochenartigen Material (R) ermöglichen, wobei die Trägerstruktur (4) ein Verriegelungsloch (42) aufweist, das durch deren Dicke geht, wobei das Verriegelungsloch (42) um eine Lochachse (43) zentriert ist und durch eine Lochkontur (44) umgrenzt ist, von der mindestens ein Teil mit einem Lochgewinde (45) versehen ist, wobei das Verriegelungsloch (42) dazu ausgelegt ist, von dem mindestens einen Befestigungsstift (2) durchquert zu werden, und
(b) ein Spannmittel (5) aufweisen, das dazu ausgelegt ist, in das Verriegelungsloch (42) eingebracht zu werden und an einem Teil des mindestens einen Befestigungsstifts (2) anzuliegen, um dessen Verriegelung in der Position sicherzustellen,
wobei das Spannmittel (5) aus einer um eine Mutterachse (51) zentrierten Verriegelungsmutter (5) besteht, wobei die Verriegelungsmutter (5) durch - einen oberen Mutterteil (52), der mit einem Profil (58) versehen ist, das für dessen Betätigung durch Drehen mittels eines geeigneten Werkzeugs ausgelegt ist, - einen unteren Mutterteil (53), der von dem oberen Mutterteil (52) abgewandt gelegen ist, - eine äußere Umfangshülle (54) mit einem Muttergewinde (55), das zum Zusammenwirken mit dem Lochgewinde (45) des Verriegelungslochs (42) ausgelegt ist, und - eine innere Kontur (56), die einen axialen Durchgang (57) umgrenzt, der für das Hindurchführen des mindestens einen Befestigungsstifts (2) ausgelegt ist, umgrenzt ist,
**dadurch gekennzeichnet, daß** die Verriegelungsmutter (5) außerdem eine verformbare Spannstruktur (59) aufweist, die dazu ausgelegt ist, sich zu verformen, wenn die Verriegelungsmutter (5) in das Lochgewinde (45) eingeschraubt wird und um an einem Teil des mindestens einen im axialen Durchgang (57) befindlichen Befestigungsstifts (2) anzuliegen.

2. Osteosynthesevorrichtung gemäß Anspruch 1, **dadurch gekennzeichnet, daß** die Spannstruktur (59) mindestens eine am unteren Mutterteil (53) befindliche Spannkralle (60) aufweist.

3. Osteosynthesevorrichtung gemäß Anspruch 2, **dadurch gekennzeichnet, daß** die mindestens eine Spannkralle (60) durch Falten um eine zur Mutterachse (51) senkrechte Faltachse (62) verformbar ist.

4. Osteosynthesevorrichtung gemäß einem der Ansprüche 2 oder 3, **dadurch gekennzeichnet, daß** die Spannstruktur (59) eine Anzahl über den Umfang des unteren Mutterteils (53) gleichmäßig verteilte Spannkrallen (60) aufweist, wobei jede der Spannkrallen (60) durch Falten um eine zur Mutterachse (51) senkrechte Faltachse (62) verformbar ist, wobei die verschiedenen Faltachsen (62) der Spannkrallen (60) in ein und derselben zur Mutterachse (51) senkrechten Ebene liegen.

5. Osteosynthesevorrichtung gemäß einem der Ansprüche 2 bis 4, **dadurch gekennzeichnet, daß** die Spannstruktur (59) zwei bis vier, vorzugsweise drei Spannkrallen (60) aufweist.

6. Osteosynthesevorrichtung gemäß einem der Ansprüche 2 bis 5, **dadurch gekennzeichnet, daß** die Spannstruktur (59) eine Anzahl Spannkrallen (60) aufweist, die durch Schlitze (61) voneinander getrennt sind, die sich in zur Mutterachse (51) radialen Ebenen erstrecken.

7. Osteosynthesevorrichtung gemäß einem der Ansprüche 1 bis 6, **dadurch gekennzeichnet, daß** die Lochkontur (44) des Verriegelungslochs (42) der Trägerstruktur (4) im Wesentlichen die Form eines Kegelstumpfs aufweist, wobei der Winkel (X) des Kegelstumpfs mit Bezug auf die Lochachse (43) des Verriegelungslochs (42) zwischen 5° und 15° beträgt.

8. Osteosynthesevorrichtung gemäß Anspruch 7, **dadurch gekennzeichnet, daß** der Winkel (X) des Kegelstumpfs mit Bezug auf die Lochachse (43) des Verriegelungslochs (42) zwischen 9° und 11° beträgt.

9. Osteosynthesevorrichtung gemäß einem der Ansprüche 1 bis 8, **dadurch gekennzeichnet, daß** die äußere Umfangshülle (54) der das Spanmittel bildenden Verriegelungsmutter (5) im Wesentlichen die Form eines Kegelstumpfs aufweist, wobei der Winkel (Y) des Kegelstumpfs mit Bezug auf die Mutterachse (51) der Verriegelungsmutter (5) zwischen 4° und 12° beträgt.

10. Osteosynthesevorrichtung gemäß Anspruch 9, **dadurch gekennzeichnet, daß** der Winkel (Y) des Kegelstumpfs mit Bezug auf die Mutterachse (51) der Verriegelungsmutter (5) zwischen 7° und 9° beträgt.

11. Osteosynthesevorrichtung gemäß einem der Ansprüche 1 bis 10, **dadurch gekennzeichnet, daß** die Mittel (41), die die Befestigung der Trägerstruktur (4) auf dem knochenartigen Material (R) ermöglichen, aus mindestens einem auf der Trägerstruktur (4) angeordneten und mit einer Befestigungsschraube (412) zusammenwirkenden Befestigungsloch (411) bestehen.

12. Osteosynthesevorrichtung gemäß Anspruch 11, **dadurch gekennzeichnet, daß** die Trägerstruktur (4) in Form einer Osteosyntheseplatte (4) vorliegt, die eine Anzahl Befestigungslöcher (411) und eine Anzahl Verriegelungslöcher (42), von denen mindestens einige für eine Aufnahme eines Befestigungsstifts (2) ausgelegt sind, aufweist.

13. Osteosynthesevorrichtung gemäß Anspruch 12, **dadurch gekennzeichnet, daß** die Befestigungslöcher (411) und die Verriegelungslöcher (42) der Osteosyntheseplatte (4) identisch sind, damit jedes wahlweise eine Befestigungsschraube (412) oder einen Befestigungsstift (2) aufnehmen kann.

## Claims

1. An osteosynthesis device comprising at least one fixation pin (2) suitable for being implanted in a bone material (R) so as to ensure an at least partial reduction of a bone fracture,
said osteosynthesis device comprising locking means (3) designed to lock in position said at least one fixation pin (2) implanted into said bone material (R),
said locking means (3) comprise:
(a) a support structure (4) provided with means (41) for its fixation to the bone material (R), said support structure (4) comprising a locking aperture (42) through its thickness, said locking aperture (42) being centred on an aperture axis (43) and being delimited by an aperture contour (44), at least part of which is provided with an aperture thread (45),said locking aperture (42) being adapted to be passed through by said at least one fixation pin (2), and
(b) a tightening means (5) designed to be fitted into said locking aperture (42) and to come into abutment against a part of said at least one fixation pin (2) in order to ensure its locking in position,
said tightening means (5) consists of a locking nut (5) centred on a nut axis (51), said locking nut (5) being delimited by - an upper nut part (52) provided with a recess (58) adapted for its driving into rotation by means of a suitable tool, - a lower nut part (53) located at the opposite of said upper nut part (52), - an outer peripheral envelope (54) comprising a nut thread (55) adapted to cooperate with said aperture thread (45) of said locking aperture (42), and - an inner contour (56) delimiting an axial hole (57) adapted for the passage of said at least one fixation pin (2),
**characterized in that** said locking nut (5) still comprising a deformable tightening structure (59) adapted to be deformed when said locking nut (5) is screwed into said aperture thread (45) and to come into abutment against a part of said at least one fixation pin (2) housed within said axial hole (57).

2. The osteosynthesis device according to claim 1, **characterized in that** said tightening structure (59) comprises at least one tightening tab (60) arranged at said lower nut part (53).

3. The osteosynthesis device according to claim 2, **characterized in that** said at least one tightening tab (60) is deformable by bending about a bending axis (62) perpendicular to said nut axis (51).

4. The osteosynthesis device according to any one of claims 2 or 3, **characterized in that** said tightening structure (59) comprises a plurality of tightening tabs (60) regularly spaced apart over the periphery of said lower nut part (53), each of said tightening tabs (60) being deformable by bending about a bending axis (62) perpendicular to said nut axis (51), the different bending axes (62) of said tightening tabs (60) being located in a same plane perpendicular to said nut axis (51).

5. The osteosynthesis device according to any one of claims 2 to 4, **characterized in that** said tightening structure (59) comprises 2 to 4 tightening tabs (60), preferably 3.

6. The osteosynthesis device according to any one of claims 2 to 5, **characterized in that** said tightening structure (59) comprises a plurality of tightening tabs (60) separated from each other by slots (61) extending in radial planes with respect to said nut axis (51).

7. The osteosynthesis device according to any one of claims 1 to 6, **characterized in that** the aperture contour (44) of the locking aperture (42) of the support structure (4) has a generally frustoconical shape, the angle (X) of said truncated cone with respect to the aperture axis (43) of said locking aperture (42) being between 5° and 15°.

8. The osteosynthesis device according to claim 7, **characterized in that** the angle (X) of said truncated cone with respect to the aperture axis (43) of said locking aperture (42) is between 9° and 11°.

9. The osteosynthesis device according to any one of claims 1 to 8, **characterized in that** the outer peripheral envelope (54) of the locking nut (5) forming said tightening means (5) has a generally frustoconical shape, the angle (Y) of said truncated cone with respect to the nut axis (51) of said locking nut (5) being between 4° and 12°.

10. The osteosynthesis device according to claim 9, **characterized in that** the angle (Y) of said truncated cone with respect to the nut axis (51) of said locking nut (5) is between 7° and 9°.

11. The osteosynthesis device according to any one of claims 1 to 10, **characterized in that** said means (41) that allow the fixation of the support structure (4) to the bone material (R) consist of at least one fixation aperture (411) arranged on said support structure (4), cooperating with a fixation screw (412).

12. The osteosynthesis device according to claim 11, **characterized in that** said support structure (4) is in the form of an osteosynthesis plate (4) comprising a plurality of fixation apertures (411) and a plurality of locking apertures (42) at least some of which are adapted to receive a fixation pin (2).

13. The osteosynthesis device according to claim 12, **characterized in that** said fixation apertures (411) and said locking apertures (42) of the osteosynthesis plate (4) are identical so as to each accommodate either a fixation screw (412) or a fixation pin (2).
